# EUROPÄISCHE PATENTSCHRIFT

(11) **EP 2 576 497 B1**
(45) Veröffentlichungstag und Bekanntmachung des Hinweises auf die Patenterteilung: **23.04.2014**
(21) Anmeldenummer: 11718345.9
(22) Anmeldetag: 02.05.2011
(51) Int. Cl.: C07C 213/06, C07C 219/08

(54) **QUARTÄRE DIALKANOLAMINESTER**
QUATERNARY DIALKANOLAMINE ESTERS
ESTERS DE DIALCANOLAMINE QUATERNAIRES

(30) Priorität: 02.06.2010 DE 102010029606
(43) Veröffentlichungstag der Anmeldung: 10.04.2013
(73) Patentinhaber: Evonik Degussa GmbH, 45128 Essen (DE)
(72) Erfinder: HERRWERTH, Sascha, 45134 Essen (DE); GRUENING, Burghard, 45134 Essen (DE); KÖHLE, Hans-Jürgen, 63533 Mainhausen (DE); ULRICH-BREHM, Isabella, 40878 Ratingen (DE)
(86) Internationale Anmeldenummer: PCT/EP2011/056924
(87) Internationale Veröffentlichungsnummer: WO 2011/151119

(56) Entgegenhaltungen:
- DE-C1- 4 308 794
- JP-A- 6 228 877

## Beschreibung

### Gebiet der Erfindung

Gegenstand der vorliegenden Erfindung sind neue quartäre Ammoniumverbindungen vom Typ der Esterquats, sowie ein Verfahren zu deren Herstellung, als auch die Verwendung dieser in Formulierungen.

### Stand der Technik

In der Vergangenheit konnten quartäre Ammoniumverbindungen ("Quats") in Form von Mono- und Dialkylquats mit einer bzw. zwei langen Alkylketten im Molekül weitverbreitete Nutzung in verschiedenen Anwendungsgebieten finden. Dazu gehören Weichspüler für Textilien, antistatische Zubereitungen, Reinigungsmittel, Hautpflegemittel, Haarkonditionierer sowie Korrosionsinhibitoren, Druckfarbenentferner für Papier und vieles mehr.
Im Bereich der Haarpflegemittel werden als Monoalkylquats beispielsweise das (INCI) Cetrimonium Chloride (z. B. kommerziell erhältlich als VARISOFT^{®} 300, Evonik Goldschmidt GmbH) und das (INCI) Behentrimonium Chloride (z. B. kommerziell erhältlich als VARISOFT^{®} BT 85 Pellets, Evonik Goldschmidt GmbH) sowie als Dialkylquat das (INCI) Dicetyldimonium Chloride (z. B. kommerziell erhältlich als VARISOFT^{®} 432 PPG, Evonik Goldschmidt GmbH) in der Industrie im großen Umfang verwendet.
Neben ihren guten Eigenschaften als Konditionierer weisen diese Alkylammonium-Kationen allerdings auch einige Nachteile auf. Dazu zählen die unzureichende biologische Abbaubarkeit und ungünstige ökotoxikologische Eigenschaften.

Besonders auf dem Gebiet der Haut- und Haarpflegemittel konnten sich neben den konventionellen Alkylquats im Laufe der Jahre außerdem die sogenannten Esterquats etablieren, beispielsweise (INCI) Distearoylethyl Dimonium Chloride, kommerziell erhältlich als VARISOFT^{®} EQ 65 Pellets (Evonik Goldschmidt GmbH) oder (INCI) Distearoylethyl Hydroxyethylmonium Methosulfate, kommerziell erhältlich als Dehyquart F 75 (Cognis).
Unter dem Oberbegriff Esterquat versteht man im allgemeinen mit Fettsäuren veresterte Di- oder Trialkanolamine in ihrer Salzform.
Diese quaternierten Fettsäurealkanolaminester-Salze stellen bekannte Stoffe dar, die nach einschlägigen Methoden der präparativen organischen Chemie erhalten werden können. Üblicherweise beruht die Herstellung der Esterquats auf einem mehrstufigen Prozess, bei dem zunächst durch Umsetzung eines Alkanolamins mit Carbonsäuren oder entsprechenden Derivaten das veresterte tertiäre Alkanolamin hergestellt wird, das dann im Anschluss mit einem geeigneten Reagens quaterniert wird.
In diesem Zusammenhang sei auf die EP0483195 verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Die dort aufgeführten Verbindungen dienen als Weichmacher für Textilien.
In der DE4308794 wird die Herstellung fester Esterquats beschrieben, indem die Quaternisierung der Triethanolaminester in Gegenwart geeigneter Dispergiermittel, bevorzugt Fettalkohole, durchgeführt wird.
Übersichten zu dieser Thematik lassen sich zum Beispiel unter R. Puchta et al. in Tens. Surf. Det., 30, 186 (1993), M. Brock in Tens. Surf. Det., 30, 394 (1993), R. Lagerman et al. in J. Am. Chem. Soc., 71, 97 (1994) oder unter I. Shapiro in Cosm. Toil., 109, 77 (1994) nachlesen.

Esterquats weisen verglichen mit Alkylquats ein günstigeres ökotoxikologisches Verhaltensprofil auf und verfügen aufgrund der vorhandenen Esterbindung auch über eine verbesserte biologische Abbaubarkeit als Alkylquats.
Die Umweltverträglichkeit von Zubereitungen bzw. deren Inhaltsstoffen gewinnt immer mehr an Bedeutung, zum einen aufgrund entsprechender gesetzlicher Regelungen, vor allem aber auch durch ein verändertes Verbraucherbewusstsein. Der Verbraucher legt immer größeren Wert auf Produkte, die sich nicht nachteilig auf die Umwelt auswirken. Dadurch rücken Eigenschaften wie Ökotoxizität oder biologische Abbaubarkeit immer mehr in den Fokus der Öffentlichkeit. Eine gute biologische Abbaubarkeit gewährleistet die Umwandlung der Stoffe durch biologische Prozesse, die üblicherweise in der Abwasseraufbereitung ablaufen. Die dabei entstehenden Fragmente weisen ein noch geringeres Risiko einer Umweltschädigung als die Ausgangsprodukte auf und sind in den herkömmlichen Verfahren der Aufbereitung von Abwasser und Feststoffabfall sehr einfach zu handhaben. Bei unzureichender biologischer Abbaubarkeit kann im Laufe der Zeit die Funktionsfähigkeit aquatischer Lebensgemeinschaften beeinträchtigt werden.
Zahlreiche der momentan auf dem Markt verfügbaren Weichmacher oder Konditioniermittel, deren biologische Abbaubarkeit bereits verbessert werden konnte, fallen jedoch in der Wirksamkeit im Vergleich zu den konventionellen Produkten ab.
Beispielsweise sind die Applikationseigenschaften der zur Zeit erhältlichen Esterquats zwar ebenfalls auf einem hohen Niveau, können bei längeren Ausspülzeiten jedoch nicht mit den Applikationseigenschaften von (INCI) Behentrimonium Chloride mithalten. Die gewünschten konditionierenden Eigenschaften werden bei den Esterquats des Standes der Technik durch den Ausspülprozess schneller reduziert.
Da man bei herkömmlichen Produkten bisher entweder auf eine effektive Leistung oder auf eine gute Umweltverträglichkeit verzichten muss, besteht nach wie vor ein hoher Bedarf an Verbindungen, die beide Bereiche abdecken, indem sie hervorragende Eigenschaften als Pflegeprodukte und eine hohe Umweltverträglichkeit in sich vereinen.

Im Bereich der Haarpflege spielen die Konditioniermittel eine wichtige Rolle. Ihre Aufgabe besteht darin, das Haar zu schützen und bereits vorhandene Schäden zu beheben. Viele Haarschädigungen entstehen dadurch, dass das Haar tagtäglich verschiedenen umweltbedingten Stressfaktoren ausgesetzt wird, dazu gehören Temperatur, Feuchtigkeit, Sonnenlicht oder Luftverschmutzung. Zusätzlich führen chemische Haarbehandlungen wie Bleichen, Färben, Tönen, Glätten oder der Einsatz von Dauerwelle zu negativen Auswirkungen auf die Haarstruktur.
So wird zum Beispiel bei einer Dauerwelle sowohl der Cortex als auch die Cuticula des Haares angegriffen. Die DisulfidBrücken des Cystins werden durch den Reduktionsschritt aufgebrochen und im anschließenden Oxidationsschritt zum Teil zu Cysteinsäure oxidiert.
Beim Bleichen wird nicht nur das Melanin zerstört, sondern es werden außerdem ca. 15 bis 25 % der Disulfid-Bindungen des Cystins bei einer milden Bleiche oxidiert. Bei einer exzessiven Bleichung können es sogar bis zu 45 % sein (K. F. de Polo, A Short Textbook of Cosmetology, 2000, Verlag für chemische Industrie, H. Ziolkowsky GmbH).

Und selbst das tägliche Waschen, Kämmen und Fönen der Haare hinterlässt Spuren. Die unerwünschten Konsequenzen zeigen sich zum Beispiel in einer rauen Haaroberfläche, einer reduzierten mechanischen Stabilität und einem Anstieg der elektrostatischen Ladung. Haarbruch, die Bildung von "Spliss", erschwerte Kämmbarkeit und der Verlust des natürlichen Glanzes sind die Folge.
Konditioniermittel haben die Aufgabe, das Haar vor diesen negativen Auswirkungen zu schützen, sollten sich dabei jedoch wenn möglich nicht negativ auf die Umwelt auswirken.

Dazu muss das Konditioniermittel in der Lage sein, auf das Haar aufzuziehen. Aufgrund der anionischen Zentren auf der Haaroberfläche, basierend auf Proteinstrukturen, bilden kationische Verbindungen eine bevorzugte Produktklasse. unter den konditionierenden Agenzien, da sie die nötige Affinität zum Haar gewährleisten. Im Falle einer bereits vorliegenden Haarschädigung ist die Zahl der anionischen Gruppen sogar noch erhöht, und zwar aufgrund der Gegenwart oxidierter Proteinstrukturen, wie z. B. der oben genannten Cysteinsäure. Gerade in diesen geschädigten Bereichen können kationische Konditioniermittel ihre volle Wirksamkeit entfalten.

Aufgabe der Erfindung war es, mindestens einem Nachteil des Standes der Technik abzuhelfen oder diesen zumindest zu verringern und insbesondere Wirkstoffe bereitzustellen, die auch bei langen Spülzeiten auf der Zieloberfläche verbleiben und ihre Wirkung weiterhin entfalten.

### Beschreibung der Erfindung

Überraschenderweise wurde gefunden, dass die im Folgenden beschriebenen Verbindungen der allgemeinen Formel (I) gemäß Anspruch 1, ein Verfahren zu deren Herstellung sowie die beschriebenen Zusammensetzungen und Formulierungen die gestellte Aufgabe zu lösen vermögen.

Gegenstand der vorliegenden Erfindung sind daher spezielle quartäre Dialkanolaminester.
Ein weiterer Gegenstand der Erfindung ist ein Verfahren zu deren Herstellung sowie deren Verwendung in Formulierungen. Ein Vorteil der vorliegenden Erfindung ist, dass die erfindungsgemäßen Verbindungen bereits bei geringen Einsatzmengen eine gute Wirkung entfalten.
Ein weiterer Vorteil ist, dass die erfindungsgemäßen Verbindungen in ökologischer Hinsicht wenig belastend sind. Noch ein Vorteil ist es, dass die erfindungsgemäßen Verbindungen auf keratinischen Fasern einen verbesserten konditionierenden Effekt bei längeren Abspülzeiten zeigen als bisher bekannte quartäre Esterverbindungen.

Einen Beitrag zur Lösung der eingangs gestellten Aufgabe leisten somit quartäre Dialkanolaminester der allgemeinen Formel (I) 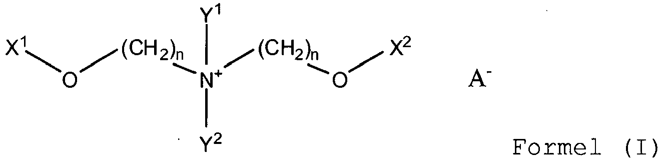 mit
Y¹ und Y² unabhängig voneinander, gleich oder verschieden ein Alkylrest mit 1 bis 6 Kohlenstoffatomen, ein Benzylrest oder H,
n = 1 bis 4,
A- ein Anion, bevorzugt ausgewählt aus der Gruppe umfassend, bevorzugt bestehend aus, Chlorid, Bromid, Iodid, Methylsulfat, Tosylat, Phosphat, Sulfat, Ethylsulfat, Hydrogensulfat, Lactat, Acetat und Citrat, bevorzugt Chlorid und Methylsulfat,
X¹ und X² unabhängig voneinander, gleich oder verschieden ausgewählt aus den Gruppen (a), (b), (c) und (d), wobei diese Gruppen bestehen aus
   (a) Acylreste enthaltend 6 bis 14 Kohlenstoffatome,
   (b) Acylreste enthaltend 15 bis 20 Kohlenstoffatome und mindestens eine Kohlenstoff-Kohlenstoff-Doppelbindung,
   (c) Acylreste enthaltend 20 bis 24 Kohlenstoffatome und
   (d) Acylreste enthaltend 3 bis 28 Kohlenstoffatome, welche nicht in den Gruppen (a) bis (c) enthalten sind, oder H, mit den Maßgaben, dass im Zahlenmittel pro Molekül der allgemeinen Formel (I)
0,30 bis 1,50, bevorzugt 0,35 bis 1,00, insbesondere 0,40 bis 0,90, Reste der Gruppe (a),
0,10 bis 1,30, insbesondere 0,20 bis 0,80, Reste der Gruppe (b),
0,40 bis 1,60, bevorzugt 0,50 bis 1,50, insbesondere 0,80 bis 1,40, Reste der Gruppe (c) und
0 bis 0,20, bevorzugt 0,0010 bis 0,10, besonders bevorzugt 0,0050 bis 0,02, Reste der Gruppe (d)
enthalten sind und
die Zahlenmittel pro Molekül der Reste der Gruppen (a), (b), (c) und (d) sich zu 2 addieren.

Es ist dem Fachmann offenbar, dass durch die allgemeine Formel (I) eine Mischung verschiedener quartärer Dialkanolaminester wiedergegeben wird.

Der Rest bestimmend Y¹ und Y² ist bevorzugt ein linearer Alkylrest, welcher bevorzugt unsubstituiert ist, ein Benzylrest oder H; insbesondere ist der Rest ausgewählt aus der Gruppe umfassend, bevorzugt bestehend aus, -CH₃, -C₂H₅, Benzyl und H.
n ist insbesondere bevorzugt gleich 2.
Die Acylreste der Gruppen (a) und (c) sind bevorzugt gesättigt.
Die Acylreste der Gruppen (a), (b), (c) und (d) sind insbesondere solche der natürlich vorkommenden Fettsäuren mit entsprechenden Kettenlängen.
Bevorzugt enthalten sie somit geradzahlige Anzahlen an Kohlenstoffatomen.
Acylreste der Gruppe (a) sind bevorzugt ausgewählt aus C₅H₁₁CO-, C₇H₁₅CO-, C₉H₁₉CO-, C₁₁H₂₃CO- und C₁₃H₂₇C- .
Da der Einsatz technischer Laurinsäure besonders bevorzugt ist, ist ein besonders bevorzugter Acylrest der Gruppe (a) ein Acylrest mit einer Kettenlänge von 12 Kohlenstoffatomen. In diesem Zusammenhang ist es bevorzugt, dass den Gruppen (b), (c) und (d) entsprechend zugeordnet werden 0 bis 1 Gew.-%, bevorzugt 0,02 bis 0,50 Gew.-% Acylreste einer Fettsäure mit einer Kettenlänge kleiner 11, bevorzugt von 6 bis 10 Kohlenstoffatomen und 0 bis 1 Gew.-%, bevorzugt 0,30 bis 0,70 Gew.-% Acylreste einer Fettsäure mit einer Kettenlänge von größer 13, bevorzugt 14 bis 28 Kohlenstoffatomen, wobei sich die Gew.-% auf die Gesamtmenge aller Acylreste bezieht, die sich aus der technischen Laurinsäure ableiten lassen.

Acylreste der Gruppe (b) sind bevorzugt ausgewählt aus C₁₅H₂₉CO-, C₁₇H₃₃CO-, C₁₇H₃₁CO-, C₁₇H₂₉CO-, C₁₉H₃₇CO-, C₁₉H₃₁CO-und C₁₉H₂₉CO-, sowie aus den Acylresten der einfach ungesättigten Fettsäuren, insbesondere der Palmitoleinsäure, Petroselinsäure, Ölsäure, Elaidinsäure, Vaccensäure, Gadoleinsäure und Icosensäure, sowie aus den Acylresten der mehrfach ungesättigten Fettsäuren, insbesondere Linolsäure, α-/γ-Linolensäure, Calendulasäure, Punicinsäure und Arachidonsäure.
Besonders bevorzugte Acylreste der Gruppe (b) bilden die Acylreste einer technischen Ölsäure mit entsprechenden Kettenlängen, insbesondere die Reste C₁₅H₂₉CO-, C₁₇H₃₃CO, C₁₇H₃₁CO-, C₁₇H₂₉CO- und C₁₉H₃₇CO- in einem Gewichtsverhältnis von 3-7 zu 68-76 zu 5-13 zu 1-3 zu 0-2. In diesem Zusammenhang ist es bevorzugt, dass den Gruppen (a), (c) und (d) entsprechend zugeordnet werden 0 bis 3 Gew.-%, bevorzugt 0,50 bis 1,50 Gew.-% Acylreste einer Fettsäure mit einer Kettenlänge von 14 Kohlenstoffatomen, 2 bis 7 Gew.-%, bevorzugt 4 bis 6 Gew.-% Acylreste einer Fettsäure mit einer Kettenlänge von 16 Kohlenstoffatomen und 0 bis 3 Gew.-%, bevorzugt 0,50 bis 1,50 Gew.-% Acylreste einer Fettsäure mit einer Kettenlänge von 18 Kohlenstoffatomen, wobei sich die Gew.-% auf die Gesamtmenge aller Acylreste bezieht, die sich aus der technischen Ölsäure ableiten lassen.

Acylreste der Gruppe (c) sind bevorzugt ausgewählt aus C₁₉H₃₉CO-, C₂₁H₄₃CO- und C₂₃H₄₇CO-.
Besonders bevorzugte Acylreste der Gruppe (c) bilden die Acylreste einer technischen Behensäure, mit entsprechenden Kettenlängen, insbesondere die Reste C₁₉H₃₉CO-, C₂₁H₄₃CO-, C₂₃H₄₇CO- in einem Gewichtsverhältnis von 4-8 zu 85-99 zu 0-3. In diesem Zusammenhang ist es bevorzugt, dass den Gruppen (a), (c) und (d) entsprechend zugeordnet werden 0 bis 3 Gew.-%, bevorzugt 0,50 bis 1,50 Gew.-% Acylreste einer Fettsäure mit einer Kettenlänge von kleiner 19, bevorzugt 6 bis 18, insbesondere 15 bis 18 Kohlenstoffatomen, 0 bis 1 Gew.-%, bevorzugt 0,25 bis 0,75 Gew.-% Acylreste einer Fettsäure mit einer Kettenlänge von 26 Kohlenstoffatomen, wobei sich die Gew.-% auf die Gesamtmenge aller Acylreste bezieht, die sich aus der technischen Behensäure ableiten lassen.

Die Acyleste der Gruppe (d) werden bevorzugt bestimmt über Carbonsäuren, welche zusätzlich in technischen Mischungen der Carbonsäuren enthalten sind, welche den Acylrest der Gruppen (a), (b) und (c) bestimmen, wobei diese technischen Mischungen zur Herstellung der erfindungsgemäßen Esterquats eingesetzt wurden. Beispielsweise enthält technische Ölsäure (Gruppe (b)) noch einen geringen Anteil gesättigter Fettsäuren mit einer Kettenlänge von 18 C-Atomen, deren Acylreste dann der Gruppe (d) zuzuordnen sind.

Es ist erfindungsgemäß bevorzugt, dass in den erfindungsgemäßen quartären Dialkanolaminestern der allgemeinen Formel (I) das molare Verhältnis der Summe von Acylresten der Gruppe (a) und der Gruppe (b) zu Acylresten der Gruppe (c) im Zahlenmittel 1 zu 0,67 bis 4, insbesondere 1 zu 0,8 bis 3 beträgt.

Ganz besonders bevorzugt sind Esterquats, bei denen die Acylreste der Gruppen (a) bis (d) bestimmt werden über die Acylreste einer Mischung bestehend aus
technischer Laurinsäure, insbesondere EDENOR C1298-100 (Emery Oleochemicals),
technischer Ölsäure, insbesondere EDENOR Ti05 (Emery Oleochemicals) und
technischer Behensäure, insbesondere EDENOR C2285 (Emery Oleochemicals).

Ein weiterer Gegenstand der vorliegenden Erfindung ist ein Verfahren zur Herstellung der erfindungsgemäßen quartären Dialkanolaminester.
Die erfindungsgemäßen quartären Dialkanolaminester sind vorteilhaft durch das im Folgenden beschriebene Verfahren erhältlich.
Das erfindungsgemäße Verfahren zur Herstellung quartärer Dialkanolaminester umfasst die Verfahrensschritte
A) Umsetzung, bevorzugt Veresterung, eines Alkyldialkanolamins, bei dem die beiden Alkanolgruppen ausgewählt sind aus der Gruppe bestehend aus, -CH₂OH, - C₂H₄OH, -C₃H₆OH und -C₄H₈OH,- insbesondere -C₂H₄OH, und die Alkylgruppe ausgewählt ist aus der Gruppe bestehend aus Alkylresten mit 1 - 6 Kohlenstoffatomen, insbesondere linearen Alkylresten, welche bevorzugt unsubstituiert sind, mit einem Gemisch aus Carbonsäuren bestehend aus den Gruppen,
   (e) Carbonsäure enthaltend 6 bis 14 Kohlenstoffatome,
   (f) Carbonsäure enthaltend 15 bis 20 Kohlenstoffatome und mindestens eine Kohlenstoff-Kohlenstoff-Doppelbindung und
   (g) Carbonsäure enthaltend 20 bis 24 Kohlenstoffatome und gegebenenfalls
   (h) Carbonsäure enthaltend 3 bis 28 Kohlenstoffatome, welche nicht in den Gruppen (e), (f) und (g) enthalten sind,
B) Umsetzung, bevorzugt Quaternisierung, des Produktes aus Verfahrensschritt A) mit einem Quaternierungsmittel ausgewählt aus der Gruppe umfassend, bevorzugt bestehend aus, Dialkylsulfate, Alkylhalogenide und Benzylhalogenide und/oder
C) Neutralisierung des Reaktionsansatzes mit einer Säure, bevorzugt einer organischen Säure,
mit der Maßgabe, dass bezogen auf alle Carbonsäuren der Gruppen (e), (f), (g) und (h) 0 bis 10 Mol%, bevorzugt 0,5 bis 5 Mol% bevorzugt 0,25 bis 1,0 Mol% Carbonsäuren der Gruppe (h) eingesetzt werden.

Besonders bevorzugt in Verfahrensschritt A) eingesetztes Alkyldialkanolamin ist das Methyldiethanolamin.

Die Carbonsäuren der Gruppe (h) werden bevorzugt bestimmt über die Carbonsäuren, welche zusätzlich in technischen Mischungen der Carbonsäuren der Gruppen (e), (f) und (g) enthalten sind, die in dem erfindungsgemäßen Verfahren eingesetzt werden.

Bevorzugte in Verfahrensschritt B) eingesetzte Alkylhalogenide weisen 1 bis 6 Kohlenstoffatome auf, insbesondere bevorzugt ist hier Methylchlorid; in diesem Zusammenhang bevorzugtes Dialkylsulfat ist Dimethylsulfat.

Die Verfahrensschritte A) und B) können unter den dem Fachmann bekannten und üblichen Verfahrensbedingungen zur Veresterung bzw. Quaternierung durchgeführt werden. In Verfahrensschritt A) wird hier somit bevorzugt das Alkyldialkanolamin mit einer Mischung der beanspruchten Fettsäuren bei 140-200 °C, ggf. unter Mitverwendung eines Veresterungskatalysators, erhitzt und entstehendes Kondensationswasser kontinuierlich abdestilliert. Zur Vervollständigung der Reaktion wird ggf. Vakuum angelegt und der Verlauf der Reaktion über die Abnahme der Säurezahl verfolgt. Eine detaillierte Anleitung hierzu findet sich unter anderem in der DE4111966.
In Verfahrensschritt B) wird der Alkyldialkanolaminester bevorzugt gegebenenfalls in Lösungsmittel bei 60 - 120 °C mit dem Quaternierungsreagenz, besonders bevorzugt mit Methylchlorid, zur Reaktion gebracht. Das Quaternierungsreagenz wird in diesem Fall im Überschuss eingesetzt, der Druck wird zwischen 1-8 bar einreguliert. Nachdem das tertiäre Amin bis zum gewünschten Umsetzungsgrad abreagiert ist, wird der Überschuss an Quaternierungsreagenz destillativ entfernt. Eine detaillierte Anleitung hierzu findet sich unter anderem in EP0643128.
Die in dem erfindungsgemäßen Verfahren bevorzugt eingesetzten Carbonsäuren der Gruppen (e), (f), (g) und (h) entsprechen den die bevorzugten Acylreste der Gruppen (a), (b), (c) und (d) liefernden Carbonsäuren der erfindungsgemäßen Verbindung.
Insbesondere bevorzugt ist hier der Einsatz einer Mischung von Carbonsäuren bestehend aus technischer Laurinsäure, insbesondere EDENOR C1298-100 (Emery Oleochemicals), technischer Ölsäure, insbesondere EDENOR Ti05 (Emery Oleochemicals), und technischer Behensäure, insbesondere EDENOR C2285 (Emery Oleochemicals).

Bevorzugt in Verfahrensschritt C) eingesetzte organische Säuren sind ausgewählt aus der Gruppe bestehend aus Zitronensäure, Weinsäure, Milchsäure, Essigsäure, Ameisensäure und Glykolsäure.

Der pH-Wert wird in Verfahrensschritt C) bevorzugt auf einen pH-Wert von 3,5 bis 5,5 eingestellt, bevorzugt unter Rühren. Dieser Schritt erfolgt bevorzugt bei einer Temperatur von insbesondere 40 - 60 °C.

Es ist erfindungsgemäß bevorzugt, dass in dem erfindungsgemäßen Verfahren pro mol Alkyldialkanolamin 0,30 bis 1,50 mol, bevorzugt 0,30 bis 1,00 mol, insbesondere 0,40 bis 0,90 mol, Carbonsäure der Gruppe (e), 0,10 bis 1,30 mol, insbesondere 0,20 bis 0,80 mol, Carbonsäure der Gruppe (f) und
0,40 bis 1,60 mol, bevorzugt 0,50 bis 1,50 mol, insbesondere 0,80 bis 1,40 mol, Carbonsäure der Gruppe (g) eingesetzt werden.
Erfindungsgemäß bevorzugt in diesem Zusammenhang ist es, dass das molare Verhältnis der eingesetzten Carbonsäuren der Summe der Gruppen (e) und (f) zu der Gruppe (g) 1 zu 0,67 bis 4, insbesondere 1 zu 0,8 bis 3 beträgt.

Ein bevorzugtes erfindungsgemäßes Verfahren ist dadurch gekennzeichnet, dass pro mol Alkyldialkanolamin insgesamt Carbonsäuren der Gruppen (e), (f), (g) und gegebenenfalls (h) in einer Menge von 1,80 bis 2,5 mol, besonders bevorzugt 1,85 bis 2,2 mol eingesetzt werden.

Ein erfindungsgemäß bevorzugtes Verfahren ist dadurch gekennzeichnet, dass in Verfahrensschritt B) mindestens ein Lösungsmittel ausgewählt aus der Gruppe bestehend aus kurzkettigen Alkoholen, Fettalkoholen, Polyolen und Carbonaten eingesetzt wird.

Kurzkettige Alkohole sind in diesem Zusammenhang solche mit einer Kettenlänge von 2 bis 6 Kohlenstoffatomen, wie beispielsweise Ethanol, Butanol, Isopropanol, Hexanol,
Als Fettalkohol wird in diesem Zusammenhang ein unverzweigter oder verzweigter Monoalkohol mit einer Alkylgruppe von 8 bis 30 C-Atomen verstanden. Bevorzugte Fettalkohole sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Guerbetalkohol, Arachylalkohol, Gadoleylalkohol, Behenylalkohol und Erucylalkohol sowie deren technische Gemische, vorzugsweise technische Kokos-oder Talgfettalkohole mit 12 bis 18, vorzugsweise mit 16 bis 18 Kohlenstoffatomen.
Bevorzugt eingesetzte Alkohole sind kurzkettige, insbesondere Isopropanol.
In Verfahrensschritt B) eingesetzte Polyole sind insbesondere Ethylenglykol, Propylenglykol, Dipropylenglykol und Butylenglykol, eingesetztes Carbonat ist insbesondere Propylen- und Ethylencarbonat.

Es ist bevorzugt, dass das Lösungsmittel in einer Menge von 0,5 bis 50 Gew.-%, bevorzugt 1 bis 40 Gew.-% eingesetzt wird, bezogen auf die Summe der Gewichtsmengen von theoretisch zu erwartender quartärer Esterverbindung und Lösungsmittel.
Eine detaillierte Anleitung hierzu ist in der DE4308794 zu finden.

Ein weiterer Gegenstand der vorliegenden Erfindung ist eine Zusammensetzung enthaltend erfindungsgemäße quartäre Dialkanolaminester und/oder quartäre Dialkanolaminester erhältlich nach dem vorstehend beschriebenen erfindungsgemäßen Verfahren, mindestens ein Lösungsmittel, insbesondere ausgewählt aus kurzkettigen Alkoholen, Fettalkoholen, Polyolen und Carbonaten und optional mindestens eine Carbonsäure ausgewählt aus mindestens einer der Gruppen (e), (f), (g) und (h).
Bevorzugt besteht die erfindungsgemäße Zusammensetzung aus den vorgenannten Komponenten.

Vorzugsweise macht das Lösungsmittel 0,5 bis 50 Gew.-%, bevorzugt 1 - 40 Gew.-%, bezogen auf das Gesamtgewicht der Zusammensetzung aus.
Die in der erfindungsgemäßen Zusammensetzung enthaltenen Lösungsmittel stammen in der Regel aus dem Zusetzen von Lösungsmittel in Verfahrensschritt B) des erfindungsgemäßen Verfahrens, so dass die in diesem Zusammenhang genannten bevorzugten Lösungsmittel bevorzugt in den erfindungsgemäßen Zusammensetzungen enthalten sind.
Somit enthalten erfindungsgemäße Zusammensetzungen bevorzugt Fettalkohole, die eine Mischung aus verschiedenen Fettalkoholen ausgewählt aus Alkoholen mit einer Kettenlänge von C12 bis C24, besonders bevorzugt eine Mischung aus Cetyl- (C16) und Stearylalkohol (C18), ist. Insbesondere bevorzugt enthaltener kurzkettiger Alkohol ist Isopropanol. Bevorzugt enthaltene Polyole sind insbesondere Ethylenglykol, Propylenglykol, Dipropylenglykol und Butylenglykol, bevorzugt enthaltenes Carbonat ist insbesondere Propylencarbonat.

Vorzugsweise machen die Carbonsäuren 0 Gew.-% bis 10 Gew.-%, bevorzugt 0,01 Gew.-% - 3 Gew.-%, bezogen auf das Gesamtgewicht der Zusammensetzung aus.
Die in der erfindungsgemäßen Zusammensetzung enthaltenen Carbonsäuren stammen in der Regel aus den überschüssig zugesetzten Carbonsäuren in Verfahrensschritt A) des erfindungsgemäßen Verfahrens, so dass die in diesem Zusammenhang genannten bevorzugten Carbonsäuren bevorzugt in den erfindungsgemäßen Zusammensetzungen enthalten sind.

Ein weiterer Gegenstand der vorliegenden Erfindung ist die Verwendung der erfindungsgemäßen quartären Dialkanolaminester und/oder der quartären Dialkanolaminester erhältlich nach dem erfindungsgemäßen Verfahren und/oder mindestens einer erfindungsgemäßen Zusammensetzung zur Herstellung von Formulierungen, insbesondere von kosmetischen oder pharmazeutischen Formulierungen und Pflege- und Reinigungsformulierungen für die Anwendung im häuslichen und industriellen Umfeld. In diesem Zusammenhang sind bevorzugte kosmetische oder pharmazeutische Formulierungen insbesondere Haut- und Haarbehandlungsformulierungen, insbesondere Haarkonditionierungsformulierungen. Bevorzugte Pflege- und Reinigungsformulierungen für die Anwendung im häuslichen und industriellen Umfeld sind in diesem Zusammenhang Textilpflegemittel, wie beispielsweise Weichspüler, und Pflegemittel für harte Oberflächen, insbesondere für Fahrzeuge, Wasserfahrzeuge, Flugzeuge, Fensterscheiben und -bänke, Duschabtrennungen, Fußböden wie Teppiche, Fliesen, Laminate, Parkett, Korkfußböden, Marmor-, Stein- und Feinsteinzeugböden, Haushaltskeramiken wie WCs, Waschbecken, Bidets, Duschtassen, Badewannen, Türklinken, Armaturen, Haushaltswerkzeuge wie Waschmaschinen, Trockner, Spülmaschinen, Spülen aus Keramik oder Edelstahl, Möbel wie Tische, Stühle, Regale, Ablageflächen, Fenster, Kochgeschirr, Geschirr und Besteck, Werkzeuge wie chirurgische Instrumente, Staubsauger, Maschinen, Rohrleitungen, Tanks und Geräte für Transport, Verarbeitung und Aufbewahrung in der Lebensmittelverarbeitung, wie beispielsweise *rinse aids,* Klarspüler.
Somit sind Formulierungen, insbesondere kosmetische oder pharmazeutische Formulierungen und Pflege- und Reinigungsformulierungen für die Anwendung im häuslichen und industriellen Umfeld enthaltend erfindungsgemäße quartäre Dialkanolaminester und/oder quartäre Dialkanolaminester erhältlich nach dem erfindungsgemäßen Verfahren und/oder mindestens eine erfindungsgemäße Zusammensetzung, insbesondere in einer Menge von 0,1 bis 7 Gew.-%, bevorzugt 0,5 bis 4 Gew.-%, besonders bevorzugt 1 bis 3 Gew.-% bezogen auf die Gesamtformulierung, insbesondere wässrige Formulierungen, welche vorzugweise einen pH-Wert von 3,5 bis 5,5 aufweisen, ein weiterer Gegenstand der vorliegenden Erfindung.
Die angegebenen, in der Formulierung enthaltenen 0,1 bis 7 Gew.-%, bevorzugt 0,5 bis 4 Gew.-%, besonders bevorzugt 1 bis 3 Gew.-% werden durch Aufsummierung aller tatsächlich in der erfindungsgemäßen Formulierung enthaltenen Komponenten (erfindungsgemäße quartäre Dialkanolaminester, quartäre Dialkanolaminester erhältlich nach dem erfindungsgemäßen Verfahren und erfindungsgemäße Zusammensetzung) ermittelt.
Bevorzugte erfindungsgemäße Formulierungen enthalten keine weiteren Alkyldialkanolaminester.
Unter dem Begriff "wässrig" wird in diesem Zusammenhang ein Wassergehalt von größer 50 Gew.-%, bevorzugt größer 75 Gew.-%, bezogen auf die Gesamtformulierung verstanden.

Bevorzugte erfindungsgemäße Formulierungen sind kosmetische Haar- und Hautpflegeformulierungen, insbesondere Haarpflegeformulierungen. Erfindungsgemäß besonders bevorzugte Formulierungen sind daher Haarshampoos, Haarspülungen, Haarfestiger, Fönfestiger, Haarpflegeemulsionen, Haarkuren, Aerosolschäume, Haarfärbemittel und Fönlotionen.

Die erfindungsgemäßen Formulierungen können z.B. mindestens eine zusätzliche Komponente enthalten, ausgewählt aus der Gruppe der
Emollients,
Co-Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.
Bezüglich weiterer fakultativer Komponenten sowie der eingesetzten Mengen dieser Komponenten wird ausdrücklich auf die dem Fachmann bekannten einschlägigen Handbücher, z. B. K. Schrader, "Grundlagen und Rezepturen der Kosemtika", 2. Auflage, Seite 329 bis 341, Hüthig Buch Verlag Heidelberg, verwiesen.
Die Mengen der jeweiligen Zusätze richten sich nach der beabsichtigten Verwendung.
Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Ein weiterer Gegenstand der vorliegenden Erfindung ist die Verwendung der quartären Dialkanolaminester und/oder der quartären Dialkanolaminester erhältlich nach dem erfindungsgemäßen Verfahren und/oder mindestens einer erfindungsgemäßen Zusammensetzung und/oder mindestens einer erfindungsgemäßen Formulierung als Pflegemittel, insbesondere als Haut- und Haarpflegemittel, und/oder zur Konditionierung von Haaren, und/oder als Weichspüler. Unter dem Begriff "Pflegemittel" wird hier eine Substanz verstanden, die den Zweck erfüllt, einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern. Für letzten Punkt sei etwa ein verbesserter Haarglanz oder eine größere Elastizität des betrachteten Gegenstandes genannt.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.
Alle Konzentrationen in den Anwendungsbeispielen sind in Gewichtsprozent angegeben. Zur Herstellung der Formulierungen wurden dem Fachmann bekannte übliche Formulierungsverfahren eingesetzt.

Folgende Figuren sind Bestandteil der Beispiele:
Figur 1: Ergebnisse der Kämmkraftmessungen bei 1 min Ausspülzeit
Figur 2: Ergebnisse der Kämmkraftmessungen bei 3 min Ausspülzeit

### Beispiele:

### Beispiel 1: Herstellung der erfindungsgemäßen Verbindung 1

Eine Mischung aus 179,5 g (0,89 mol) Laurinsäure, 251,4 g (0,89 mol) Ölsäure und 619 g (1,795 mol) Behensäure werden in einem Kolben mit mechanischem Rührer, Kolonne und Destillationsbrücke bei 70 °C homogenisiert. Unter einer Stickstoffatmosphäre werden 223,5 g (1,875 mol) Methyldiethanolamin zugetropft und die Mischung langsam auf 190 °C aufgeheizt. Reaktionswasser wird kontinuierlich abdestilliert. 2 h nach Erreichen der Solltemperatur wird Vakuum angelegt und die Reaktionsmischung weitere 3 h bei 190 °C gerührt. Die Säurezahl beträgt 3,1 mg KOH/g und die Reaktionsmischung wird auf 60 °C abgekühlt.

771,4 g (1,2 mol) des oben beschriebenen Diesters werden mit 448 g Fettalkohol C 16-18 gemischt und in einem Druckgefäß bei 80 - 110 °C mit 73,5 g (1,46 mol) Methylchlorid portionsweise versetzt, so dass der Maximaldruck 5 bar nicht übersteigt. Nach 5 h wird die Reaktionsmischung analysiert, die Gesamt-Amin-Zahl beträgt 2,9 mg KOH/g, der Druck wird langsam entspannt, und der Überschuss Methylchlorid im Vakuum abdestilliert. Der Aktivgehalt des Fertigprodukts beträgt 0,93 meq/g, der Schmelzpunkt wird mit 58 °C bestimmt.

### Beispiel 2: Herstellung der erfindungsgemäßen Verbindung 2

Eine Mischung aus 60,1 g (0,3 mol) Laurinsäure, 196,0 g (0,7 mol) Ölsäure und 362,1 g ( 1,05 mol) Behensäure werden in einem Kolben mit mechanischem Rührer, Kolonne und Destillationsbrücke bei 70 °C homogenisiert. Unter einer Stickstoffatmosphäre werden 119,2 g (1,0 mol) Methyldiethanolamin zugetropft und die Mischung langsam auf 190 °C aufgeheizt. Reaktionswasser wird kontinuierlich abdestilliert. 2 h nach Erreichen der Solltemperatur wird Vakuum angelegt und die Reaktionsmischung weitere 3 h bei 190 °C gerührt. Die Säurezahl beträgt 13,6 mg KOH/g, und die Reaktionsmischung wird auf 60 °C abgekühlt.

701,0 g (1,0 mol) des oben beschriebenen Diesters werden mit 404 g Fettalkohol C 16-18 gemischt und in einem Druckgefäß bei 80 - 110 °C mit 75,8 g (1,5 mol) Methylchlorid portionsweise versetzt, so dass der Maximaldruck 5 bar nicht übersteigt. Nach 5 h wird die Reaktionsmischung analysiert, die Gesamt-Amin-Zahl beträgt 3,1 mg KOH/g, der Druck wird langsam entspannt und der Überschuss Methylchlorid im Vakuum abdestilliert. Der Aktivgehalt des Fertigprodukts beträgt 0,86 meq/g, der Schmelzpunkt wird mit 52 °C bestimmt.

### Beispiel 3: Überprüfung von Haarbehandlungsmitteln unter Verwendung der erfindungsgemäßen Verbindungen: Beispiel 1 und Beispiel 2.

Für die anwendungstechnische Beurteilung wurden Haartressen verwendet, die durch eine Bleichbehandlung standardisiert vorgeschädigt wurden. Dazu werden friseurübliche Produkte eingesetzt. Die Schädigung der Haartressen wird im Detail in der DE10327871 beschrieben.
Für die anwendungstechnische Beurteilung wurden die erfindungsgemäßen Verbindungen Beispiel 1 und Beispiel 2 in einer einfachen kosmetischen Formulierung eingesetzt.
Als Referenzverbindungen wurden das kommerziell erhältliche Alkylquat (INCI) Behentrimonium Chloride (VARISOFT^{®} BT 85 Pellets, Evonik Goldschmidt GmbH) und das kommerziell erhältliche Esterquat (INCI) Distearoylethyl Dimonium Chloride (VARISOFT^{®} EQ 65 Pellets, Evonik Goldschmidt GmbH) verwendet.
Die Anwendungseigenschaften beim Einsatz in Haarspülungen wurden in den folgenden Rezepturen überprüft (Tab. 1):

**Tab. 1: Haarspülung-Formulierungen zur Austestung der haarkonditionierenden Eigenschaften.**

| **Formulierungs-Beispiele** | **C0a** | **1a** | **2a** | **V3a** | **V4a** |
|---|---|---|---|---|---|
| TEGINACID® C, Evonik Goldschmidt GmbH (INCI: Ceteareth-25) | 0,5 % | 0,5 % | 0,5 % | 0,5 % | 0,5 % |
| TEGO® Alkanol 16, Evonik Goldschmidt GmbH (INCI: Cetyl Alcohol)) | 4 % | 4 % | 4 % | 4 % | 4 % |
| Beispiel 1 (65%-ig in C 16 Fettalkohol) | - | 2,3 % | - | - | - |
| Beispiel 2 (65%-ig in C 16 Fettalkohol) | - | - | 2,3 % | - | - |
| VARISOFT® EQ 65 Pellets, 65 %-ig in C 16 Fettalkohol, Evonik Goldschmidt GmbH (INCI: Distearoylethyl Dimonium Chloride, Cetearyl Alcohol) | - | - | - | 2,3 % | - |
| VARISOFT® BT 85, 85 %-ig in Isopropanol, Evonik Goldschmidt GmbH (INCI: Behentrimonium Chloride) | - | - | - | - | 1,76 % |
| Wasser, demineralisiert | ad 100,0 | | | | |
| Zitronensäure | ad pH 4,0 | | | | |

Die Zusammensetzung der Testformulierungen ist zur Vermeidung der Beeinflussung der Testergebnisse durch (normalerweise vorhandene) Formulierungsbestandteile bewusst einfach gewählt. Erfindungsgemäße Formulierungen können neben den genannten Inhaltsstoffen und/oder anstatt der genannten Inhaltsstoffe noch weitere Inhaltsstoffe enthalten. Insbesondere die Kombination mit weiteren Inhaltsstoffen kann bei den beschriebenen Effekten zu einer synergistischen Verbesserung führen.

Die Vorbehandlung der Haare erfolgt durch eine Shampooformulierung (Tab. 2), welche keine
Konditioniermittel enthält.

**Tab. 2: Shampooformulierung für die Vorbehandlung der Haartressen.**

| | |
|---|---|
| Texapon NSO^{®}, 28 %-ig, Cognis (INCI: Sodium Laureth Sulfate) | 42, % |
| NaCl | 3 % |
| Wasser, demineralisiert | ad 100,0 |

Standardisierte Behandlung von vorgeschädigten Haarsträhnen mit konditionierenden Proben:
Die wie oben beschrieben vorgeschädigten Haarsträhnen werden mit der Shampooformulierung aus Tab. 2 gewaschen. Hierbei werden die Haarsträhnen unter fließendem, warmem Wasser benetzt. Das überschüssige Wasser wird leicht von Hand ausgedrückt, dann wird das Shampoo aufgebracht und 1 min lang sanft ins Haar eingearbeitet (0.5 ml / 2 g Haarsträhne). Die Haartresse wird für 30 s unter fließend warmem Wasser ausgespült. Diese Prozedur wird ein weiteres mal wiederholt, nur dass abschließend 1 min lang ausgespült wird.

Anschließend werden direkt nach dem Waschen die Haarsträhnen mit den Haarspülungs-Formulierungen aus Tab. 1 konditioniert.
Hierbei wird die Spülung aufgebracht und sanft ins Haar eingearbeitet (0.5 ml / 2 g Haarsträhne). Nach einer Verweilzeit von 1 min wird das Haar für a) 1 min oder für b) 3 min gespült.

Vor der sensorischen Beurteilung wird das Haar an der Luft bei 50 % Luftfeuchtigkeit und 25 °C für mindestens 12 h getrocknet.

Beurteilungskriterien:
Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung.
Die Testkriterien sind:
Nasskämmbarkeit, Nassgriff, Trockenkämmbarkeit, Trockengriff, Aussehen/Glanz.

In den folgenden Tabellen werden die Ergebnisse der sensorischen Beurteilung der wie oben beschrieben durchgeführten Behandlung der Haarsträhnen bei a) 1 min Ausspülzeit und bei b) 3 min Ausspülzeit mit den erfindungsgemäßen Formulierungen 1a und 2a, den Vergleichsformulierungen V3a und V4a und der Kontrollformulierung C0a (Control ohne Testsubstanz) verglichen.

### a) 1 min Ausspülzeit

**Tab. 3: Ergebnisse der Konditionierung von Haar bei 1 min Ausspülzeit**

| | Nasskämmbarkeit | Nassgriff | Trockenkämmbarkeit | Trockengriff |
|---|---|---|---|---|
| Erfindungsgemäße Formulierung 1a | 4.6 | 4.7 | 4.7 | 4.6 |
| Erfindungsgemäße Formulierung 2a | 4.7 | 4.6 | 4.6 | 4.7 |
| Vergleichsformulierung V3a (nicht erfindungsgemäß) | 4.5 | 4.5 | 4.6 | 4.6 |
| Vergleichsformulierung V4a (nicht erfindungsgemäß) | 4.6 | 4.5 | 4.5 | 4.5 |
| Kontrollformulierung C0a (Placebo) | 2.0 | 1.5 | 2.8 | 3.3 |

### b) 3 min Ausspülzeit

**Tab. 4: Ergebnisse der Konditionierung von Haar bei 3 min Ausspülzeit**

| | Nasskämmbarkeit | Nassgriff | Trockenkämmbarkeit | Trockengriff |
|---|---|---|---|---|
| Erfindungsgemäße Formulierung 1a | 4.0 | 4.2 | 4.4 | 4.4 |
| Erfindungsgemäße Formulierung 2a | 4.2 | 4.1 | 4.2 | 4.3 |
| Vergleichsformulierung V3a (nicht erfindungsgemäß) | 3.5 | 3.4 | 3.4 | 3.6 |
| Vergleichsformulierung V4a (nicht erfindungsgemäß) | 3.9 | 4.0 | 4.0 | 4.2 |
| Kontrollformulierung C0a (Placebo) | 1.6 | 1.4 | 2.9 | 3.2 |

Die Ergebnisse in Tabelle 3 zeigen, dass die erfindungsgemäßen Formulierungen 1a und 2a bei 1 min Ausspülzeit sehr gute konditionierende Eigenschaften wie die Vergleichsformulierungen V3a und V4a aufweisen. Die Vergleichsformulierungen V3a und V4a haben bei 1 min Ausspülzeit erwartungsgemäß ähnlich konditionierende Eigenschaften.

Die Ergebnisse in Tabelle 4 zeigen, dass die erfindungsgemäßen Formulierungen 1a und 2a bei 3 min Ausspülzeit bessere konditionierende Eigenschaften als die Vergleichsformulierung V4a aufweisen. Die Vergleichsformulierung V4a enthält als konditionierende Verbindung VARISOFT^{®} BT 85 (85 %ig in Isopropanol, Evonik Goldschmidt GmbH, INCI: Behentrimonium Chloride), ein Alkylquat, das für seine sehr guten konditionierenden Eigenschaften auch bei langen Ausspülzeiten bekannt ist. Die Vergleichsformulierung V3a enthält als konditionierende Verbindung VARISOFT^{®} EQ 65 Pellets (65 %ig in C 16 Fettalkohol, Evonik Goldschmidt GmbH, INCI: Distearoylethyl Dimonium Chloride, Cetearyl Alcohol), ein Esterquat, das sehr gute konditionierenden Eigenschaften bei 1 min Ausspülzeit aufweist (siehe Tab. 3), bei 3 min Ausspülzeit jedoch eine deutlich schlechtere Konditionierung als die erfindungsgemäßen Esterquats Beispiel 1 und 2 hat.

### Beispiel 4: Einfluss der erfindungsgemäßen Verwindungen auf Kämmkräfte von Haaren

Versuchsbedingungen:
Messgerät: Diastron MTT 175
Messstrecke: 20 cm
Kämmgeschwindigkeit: 2000 mm/min
Verwendete Haarsträhnen: Länge = 23 cm; Breite = 1,5 cm; Gewicht = 2 g
Messbedingungen: T = 22 °C. Die Haarsträhnen werden mit einer Restfeuchte von 60 %, bestimmt durch
Gewichtsbestimmung, gemessen.

Für die Versuche wird europäisches, ungeschädigtes, dunkelbraunes Haar eingesetzt. Zur Durchführung der Kämmkraftmessungen wird dieses Haar im Labor nach Standardbedingungen durch Dauerwelle geschädigt: 1.) 4 g Dauerwell-Lsg./g Haar, 15 min. einwirken lassen, 2 min. ausspülen unter fließendem Leitungswasser (T = 35 °C). (Dauerwell-Lsg: Universal-Dauerwelle, Basler) 2.) 4 g Fixierer (1 Teil Fixierlsg. + 3 Teile Wasser)/g Haar, 10 min. einwirken lassen, 2 min. ausspülen. (Fixier-Lsg: Schaumfixierung Konzentrat, Basler)

Durchführung der Kämmkraftmessung vor der Behandlung mit der Testformulierung:
Die vorgeschädigten Haarsträhnen werden über Nacht klimatisiert.
   3.) Die Haarsträhne wird 1 min in einer Pufferlösung (Na-Citrat, pH = 6) eingetaucht.
   4.) Die Haarsträhne wird so lange von Hand vorgekämmt, bis keine Änderung des Kämmwiderstandes festzustellen ist.
   5.) Die Haarsträhne wird am Messgerät befestigt und die erste Kämmkraftmessung wird durchgeführt. Die Messung wird insgesamt 10 mal wiederholt.

Behandlung der Strähnen:
Pro Haarsträhne werden 0,5 g der jeweiligen Testformulierung verwendet (2 g Haar/0,5 g Lösung). Die Formulierung wird 30 sec. in das Haar einmassiert und dann für 5 min. liegen gelassen, anschließend 1 min. bzw. 3 min. unter fließendem Leitungswasser abgespült.

Durchführung der Kämmkraftmessung nach der Behandlung mit der Testformulierung:
Die Punkte 3-5 werden wiederholt.

Anschließend wird die Kämmarbeit (%) vor und nach der Behandlung mit der Testformulierung berechnet.

Verwendete Testformulierungen:
Die Kämmkräfte beim Einsatz in Haarspülungen wurden in den folgenden Rezepturen überprüft (Tab. 5):

**Tab. 5: Haarspülung-Formulierungen zur Austestung der haarkonditionierenden Eigenschaften.**

| **Formulierungs-Beispiele** | **C0b** | **1b** | **2b** | **V3b** | **V4b** |
|---|---|---|---|---|---|
| TEGINACID® C, Evonik Goldschmidt GmbH (INCI: Ceteareth-25) | 0,5 % | 0,5 % | 0,5 % | 0,5 % | 0,5 % |
| TEGO® Alkanol 16, Evonik Goldschmidt GmbH (INCI: Cetyl Alcohol)) | 4 % | 4 % | 4 % | 4 % | 4 % |
| Beispiel 1 (65%-ig in C 16 Fettalkohol) | - | 1,5 % | - | - | - |
| Beispiel 2 (65%-ig in C 16 Fettalkohol) | - | - | 1, % | - | - |
| VARISOFT® EQ 65 Pellets, 65 %-ig in C 16 Fettalkohol, Evonik Goldschmidt GmbH (INCI: Distearoylethyl Dimonium Chloride, Cetearyl Alcohol) | - | - | - | 1,5 % | - |
| VARISOFT® BT 85, 85 %-ig in Isopropanol, Evonik Goldschmidt GmbH (INCI: Behentrimonium Chloride) | - | - | - | - | 1,18 % |
| Wasser, demineralisiert | ad 100,0 | | | | |
| Zitronensäure | ad pH 4,0 | | | | |

In der folgenden Abbildung werden die Ergebnisse der Kämmkraftmessungen der wie oben beschrieben durchgeführten Experimente bei a) 1 min Ausspülzeit und bei b) 3 min Ausspülzeit mit den erfindungsgemäßen Formulierungen 1b und 2b, den Vergleichsformulierungen V3b und V4b und der Kontrollformulierung C0b (Control ohne Testsubstanz) verglichen.

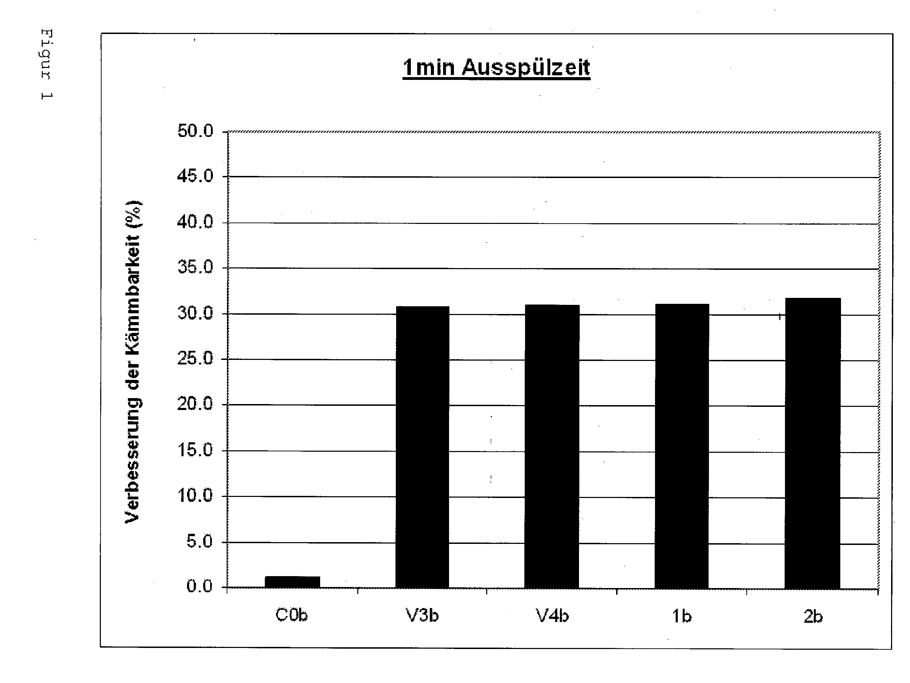

Die Ergebnisse in Figur 1 zeigen, dass die erfindungsgemäßen Formulierungen 1b und 2b bei 1 min Ausspülzeit eine ausgeprägte Reduktion der Kämmkräfte wie die Vergleichsformulierungen V3b und V4b aufweisen. Die Vergleichsformulierungen V3b und V4b generieren bei 1 min Ausspülzeit erwartungsgemäß eine ähnlich ausgeprägte Reduktion der Kämmkräfte.

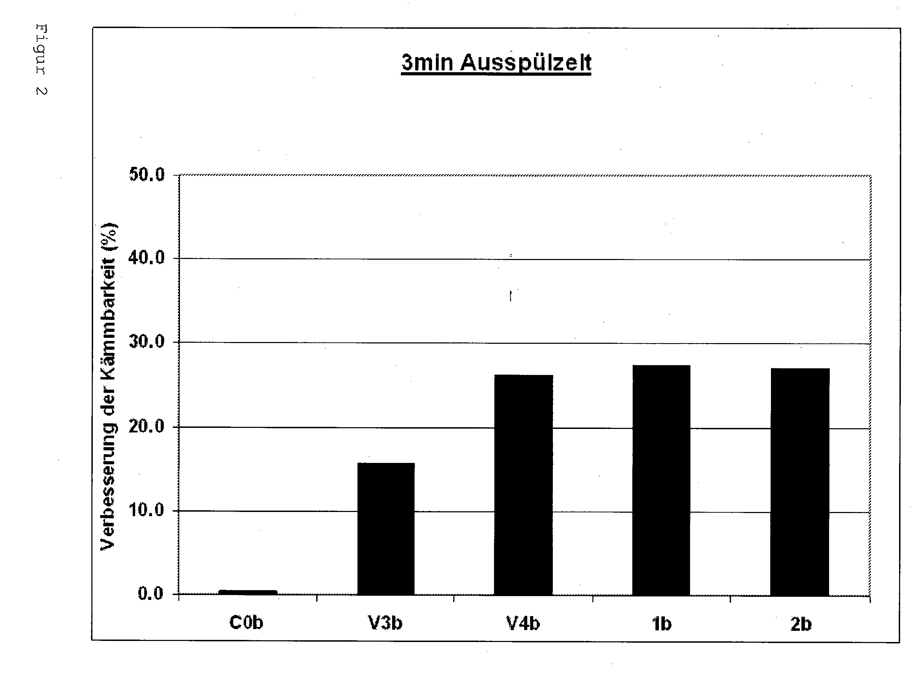

Die Ergebnisse in Figur 2 zeigen, dass die erfindungsgemäßen Formulierungen 1b und 2b bei 3 min Ausspülzeit zu einer stärkeren Reduktion der Kämmkräfte als die Vergleichsformulierung V4b aufweisen. Die Vergleichsformulierung V4b enthält als konditionierende Verbindung VARISOFT^{®} BT 85 (85 %ig in Isopropanol, Evonik Goldschmidt GmbH, INCI: Behentrimonium Chloride), ein Alkylquat, das für seine starke Reduktion der Kämmkräfte auch bei langen Ausspülzeiten bekannt ist. Die Vergleichsformulierung V3a enthält als konditionierende Verbindung VARISOFT^{®} EQ 65 Pellets (65 %ig in C 16 Fettalkohol, Evonik Goldschmidt GmbH, INCI: Distearoylethyl Dimonium Chloride, Cetearyl Alcohol), ein Esterquat, das eine gute Reduktion der Kämmkräfte bei 1 min Ausspülzeit aufweist (siehe Abb.2), bei 3 min Ausspülzeit jedoch eine deutlich schlechtere Reduktion der Kämmkräfte als die erfindungsgemäßen Esterquats Beispiel 1 und 2 hat.

### Formulierungsbeispiele:

Diese Formulierungsbeispiele zeigen, dass die erfindungsgemäßen quaternären Verbindungen in einer Vielzahl kosmetischer Formulierungen eingesetzt werden können.

### Formulierungsbeispiel 1) Pearlized Shampoo

| | |
|---|---|
| TEXAPON^{®} NSO, Cognis, 28%-ig (INCI: Sodium Laureth Sulfate) | 32,25% |
| Erfindungsgemäßes Beispiel 1 | 0,25% |
| Perfume | 0,25% |
| Water | 55,25% |
| TEGO^{®} Betain F 50, Evonik Goldschmidt GmbH, 38%-ig (INCI: Cocamidopropyl Betaine) | 8,00% |
| TEGO^{®} Pearl N 300 Evonik Goldschmidt GmbH (INCI: Glycol Distearate; Laureth-4; Cocamidopropyl Betaine) | 2,00% |
| ANTIL^{®} 171 Evonik Goldschmidt GmbH (INCI: PEG-18 Glyceryl Oleate/Cocoate) | 1,50% |
| NaCl | 0,50% |
| Preservative | q.s. |

### Formulierungsbeispiel 2) Rinse-Off Conditioner

| | |
|---|---|
| Water | 92,0% |
| TEGINACID® C, Evonik Goldschmidt GmbH (INCI: Ceteareth-25) | 0,5% |
| Erfindungsgemäßes Beispiel 1 | 2,50% |
| TEGO^{®} Alkanol 1618, Evonik Goldschmidt GmbH (INCI: Cetearyl Alcohol) | 5,00% |
| Preservative, Perfume | q.s. |

### Formulierungsbeispiel 3) Rinse-Off Conditioner

| | |
|---|---|
| Water | 91,0% |
| Erfindungsgemäßes Beispiel 2 | 2, 00% |
| VARISOFT^{®} BT 85, Evonik Goldschmidt GmbH (INCI: Behentrimonium Chloride) | 2,00% |
| TEGO^{®} Alkanol 1618, Evonik Goldschmidt GmbH (INCI: Cetearyl Alcohol) | 5,00% |
| Preservative, Perfume | q.s. |

### Formulierungsbeispiel 4) Rinse-Off Conditioner

| | |
|---|---|
| Water | 90,20% |
| Erfindungsgemäßes Beispiel 1 | 2,00% |
| VARISOFT^{®} EQ 65, Evonik Goldschmidt GmbH (INCI: Distearoyl Dimonium Chloride, Cetearyl Alcohol) | 2,00% |
| TEGO^{®} Alkanol 1618, Evonik Goldschmidt GmbH (INCI: Cetearyl Alcohol) | 5,80% |
| Preservative, Perfume | q.s. |

### Formulierungsbeispiel 5) Rinse-Off Conditioner

| | |
|---|---|
| Water | 89,20% |
| TEGINACID® C, Evonik Goldschmidt GmbH (INCI: Ceteareth-25) | 0,5% |
| VARISOFT^{®} EQ 65, Evonik Goldschmidt GmbH (INCI: Distearoyl Dimonium Chloride, Cetearyl Alcohol) | 2,00% |
| Erfindungsgemäßes Beispiel 1 | 2,00% |
| ABIL^{®} Quat 3272, Evonik Goldschmidt GmbH (INCI: Quaternium-80) | 1,30% |
| TEGO^{®} Alkanol 1618, Evonik Goldschmidt GmbH (INCI: Cetearyl Alcohol) | 5,00% |
| Preservative, Perfume | q.s. |

### Formulierungsbeispiel 6) Rinse-Off Conditioner

| | |
|---|---|
| TEGINACID^{®} C, Evonik Goldschmidt GmbH (INCI: Ceteareth-25) | 0,50% |
| TEGO^{®} Alkanol 16, Evonik Goldschmidt GmbH (INCI: Cetyl Alcohol) | 2,00% |
| TEGO^{®} Amid S 18, Evonik Goldschmidt GmbH (INCI: Stearamidopropyl Dimethylamine) | 1,00% |
| Erfindungsgemäßes Beispiel 1 | 1,50% |
| Propylene Glycol | 2,00% |
| Citric Acid Monohydrate | 0,30% |
| Water | 92,70% |
| Preservative, Perfume | q.s. |

### Formulierungsbeispiel 7) Rinse-Off Conditioner

| | |
|---|---|
| TEGINACID^{®} C, Evonik Goldschmidt GmbH (INCI: Ceteareth-25) | 0,50% |
| TEGO^{®} Alkanol 16, Evonik Goldschmidt GmbH (INCI: Cetyl Alcohol) | 5,00% |
| TEGOSOFT^{®} DEC, Evonik Goldschmidt GmbH (INCI: Diethylhexyl Carbonate) | 1,00% |
| Erfindungsgemäßes Beispiel 2 | 1,50% |
| Water | 89,20% |
| TEGO^{®} Cosmo C 100 Evonik Goldschmidt GmbH (INCI: Creatine) | 0,50% |
| Propylene Glycol | 2,00% |
| Citric Acid Monohydrate | 0,30% |
| Preservative, Perfume | q.s. |

### Formulierungsbeispiel 8) Leave-In Conditioner Spray

| | |
|---|---|
| Lactic Acid, 80% | 0,40% |
| Water | 95,30% |
| Erfindungsgemäßes Beispiel 1 | 1,20% |
| TEGIN^{®} G 1100 Pellets, Evonik Goldschmidt GmbH (INCI: Glycol Distearate) | 0,60% |
| TEGO^{®} Care PS, Evonik Goldschmidt GmbH (INCI: Methyl Glucose Sesquistearate) | 1,20% |
| TEGOSOFT^{®} DEC, Evonik Goldschmidt GmbH (INCI: Diethylhexyl Carbonate) | 1,30% |
| Preservative, Perfume | q.s. |

### Formulierungsbeispiel 9) Leave-In Conditioner Spray

| | |
|---|---|
| Lactic Acid, 80% | 0,40% |
| Water | 95,30% |
| TEGO^{®} Amid S 18, Evonik Goldschmidt GmbH (INCI: Stearamidopropyl Dimethylamine) | 1,20% |
| Erfindungsgemäßes Beispiel 1 | 0,30% |
| TEGIN^{®} G 1100 Pellets, Evonik Goldschmidt GmbH (INCI: Glycol Distearate) | 0,90% |
| TEGO^{®} Care PS, Evonik Goldschmidt GmbH (INCI: Methyl Glucose Sesquistearate) | 1,60% |
| TEGOSOFT^{®} DEC, Evonik Goldschmidt GmbH (INCI: Diethylhexyl Carbonate) | 0,30% |
| Preservative, Perfume | q.s. |

### Formulierungsbeispiel 10) Leave-In Conditioner Spray

| | |
|---|---|
| TAGAT^{®} CH-40, Evonik Goldschmidt GmbH (INCI: PEG-40 Hydrogenated Castor Oil) | 2,20% |
| Ceramide VI, Evonik Goldschmidt GmbH (INCI: Ceramide 6 II) | 0,05% |
| Perfume | 0,20% |
| Water | 90,95% |
| Erfindungsgemäßes Beispiel 1 | 0,30% |
| LACTILC^{®} Evonik Goldschmidt GmbH (INCI: Sodium Lactate; Sodium PCA; Glycine; Fructose; Urea; Niacinamide; Inositol; Sodium benzoate; Lactic Acid) | 2,00% |
| TEGO^{®} Betain F 50 Evonik Goldschmidt GmbH 38% (INCI: Cocamidopropyl Betaine) | 2,30% |
| Citric Acid (10% in water) | 2,00% |

### Formulierungsbeispiel 11) Leave-In Conditioner Foam

| | |
|---|---|
| Erfindungsgemäßes Beispiel 1 | 0, 30% |
| TAGAT^{®} CH-40, Evonik Goldschmidt GmbH (INCI: PEG-40 Hydrogenated Castor Oil) | 1,0% |
| Perfume | 0,30% |
| TEGO^{®} Betain 810, Evonik Goldschmidt GmbH (INCI: Capryl/Capramidopropyl Betaine) | 2,00% |
| Water | 94,00% |
| TEGO^{®} Cosmo C 100, Evonik Goldschmidt GmbH (INCI: Creatine) | 0,50% |
| TEGOCEL^{®} HPM 50, Evonik Goldschmidt GmbH (INCI: Hydroxypropyl Methylcellulose) | 0,30% |
| VARISOFT^{®} 300, Evonik Goldschmidt GmbH (INCI: Cetrimonium Chloride) | 1,0% |
| LACTIL^{®} Evonik Goldschmidt GmbH (INCI: Sodium Lactate; Sodium PCA; Glycine; Fructose; Urea; Niacinamide; Inositol; Sodium benzoate; Lactic Acid) | 0,50% |
| Citric Acid, 30% | 0,10% |
| Preservative | q.s. |

### Formulierungsbeispiel 12) Strong Hold Styling Gel

| | |
|---|---|
| TEGO^{®} Carbomer 141, Evonik Goldschmidt GmbH (INCI: Carbomer) | 1,20% |
| Water | 66,70% |
| NaOH, 25% | 2,70% |
| PVP/VA W-735, ISP (INCI: PVP/VA Copolymer | 16,00% |
| Erfindungsgemäßes Beispiel 1 | 0,30% |
| Alcohol Denat. | 10,50% |
| TAGAT^{®} O 2 V, Evonik Goldschmidt GmbH (INCI: PEG-20 Glyceryl Oleate) | 2,00% |
| Perfume | 0,30% |
| ABIL^{®} B 88183, Evonik Goldschmidt GmbH (INCI: PEG/PPG-20/6 Dimethicone) | 0,30% |
| Preservative | q.s. |

### Formulierungsbeispiel 13) Körperpflegemittel

| | |
|---|---|
| TEXAPON^{®} NSO, Cognis, 28%-ig (INCI: Sodium Laureth Sulfate) | 30,00% |
| TEGOSOET^{®} PC 31, Evonik Goldschmidt GmbH (INCI: Polyglyceryl-3 Caprate) | 0,70% |
| Erfindungsgemäßes Beispiel 2 | 0,30% |
| Perfume | 0,30% |
| Water | 53,90% |
| TEGOCEL^{®} HPM 4000, Evonik Goldschmidt GmbH (INCI: Hydroxypropyl Methylcellulose) | 0,30% |
| REWOTERIC^{®} AM C, Evonik Goldschmidt GmbH, 32%-ig (INCI: Sodium Cocoamphoacetate) | 10,00% |
| Citric Acid Monohydrate | 0,50% |
| REWODERM^{®} LI S 80, Evonik Goldschmidt GmbH (INCI: PEG-200 Hydrogenated Glyceryl Palmate; PEG-7 Glyceryl Cocoate) | 2,00% |
| TEGO^{®} Pearl N 300, Evonik Goldschmidt GmbH (INCI: Glycol Distearate; Laureth-4; Cocamidopropyl Betaine) | 2,00% |

### Formulierungsbeispiel 14) Mild Foam Bath

| | |
|---|---|
| TEXAPON^{®} NSO, Cognis, 28%-ig (INCI: Sodium Laureth Sulfate) | 27,00% |
| REWOPOL^{®} SB FA 30, Evonik Goldschmidt GmbH, 40%-ig (INCI: Disodium Laureth Sulfosuccinate) | 12,00% |
| TEGOSOFT^{®} LSE 65 K SOFT, Evonik Goldschmidt GmbH (INCI: Sucrose Cocoate) | 2, 00% |
| Water | 39,00% |
| REWOTERIC^{®} AM C, Evonik Goldschmidt GmbH, 32%-ig (INCI: Sodium Cocoamphoacetate) | 13,00% |
| Erfindungsgemäßes Beispiel 2 | 0,40% |
| Citric Acid (30% in water) | 3,00% |
| ANTIL^{®} 171 Evonik Goldschmidt GmbH (INCI: PEG-18 Glyceryl Oleate/Cocoate) | 1,60% |
| TEGO^{®} Pearl N 300 Evonik Goldschmidt GmbH (INCI: Glycol Distearate; Laureth-4; Cocamidopropyl Betaine) | 2,00% |

### Formulierungsbeispiel 15) Rinse-Off Conditioner

| | |
|---|---|
| Water | 89,20% |
| Erfindungsgemäßes Beispiel 2 | 3,00% |
| ABIL^{®} OSW 5, Evonik Goldschmidt GmbH (INCI: Cyclopentasiloxane; Dimethiconol) | 1,80% |
| TEGO^{®} Alkanol 1618, Evonik Goldschmidt GmbH (INCI: Cetearyl Alcohol) | 6,00% |
| Preservative, Perfume | q.s. |

### Formulierungsbeispiel 16) Rinse-Off Conditioner

| | |
|---|---|
| Water | 89,20% |
| TEGINACID® C, Evonik Goldschmidt GmbH (INCI: Ceteareth-25) | 0,5% |
| VARISOFT^{®} EQ 65, Evonik Goldschmidt GmbH (INCI: Distearyl Dimonium Chloride, Cetearyl Alcohol) | 1,50% |
| Erfindungsgemäßes Beispiel 2 | 2,00% |
| ABIL^{®} Soft AF 100, Evonik Goldschmidt GmbH (INCI: Methoxy PEG/PPG-7/3 Aminopropyl Dimethicone) | 1,00% |
| TEGO^{®} Alkanol 1618, Evonik Goldschmidt GmbH (INCI: Cetearyl Alcohol) | 5,80% |
| Preservative, Perfume | q.s. |

### Formulierungsbeispiel 17) Rinse-Off Conditioner

| | |
|---|---|
| Water | 91,50% |
| TEGINACID® C, Evonik Goldschmidt GmbH (INCI: Ceteareth-25) | 0,5% |
| Erfindungsgemäßes Beispiel 2 | 2,00% |
| SF 1708, Momentive (INCI: Amodimethicone) | 1,00% |
| TEGO^{®} Alkanol 1618, Evonik Goldschmidt GmbH (INCI: Cetearyl Alcohol) | 5,00% |
| Preservative, Perfume | q.s. |

### Formulierungsbeispiel 18) Weichspüler 1

| | |
|---|---|
| Erfindungsgemäßes Beispiel 1 | 4,00% |
| Dyestuff, 1% | 0,15% |
| Perfume | 0,20% |
| Water, demin. | 94% |

### Formulierungsbeispiel 19) Weichspüler 2

| | |
|---|---|
| Erfindungsgemäßes Beispiel 2 | 4,00% |
| Plantatex HCC | 5,00% |
| Glycerin (99,5 Gew.%) | 17,00% |
| Phenonip | 0,70% |
| Water, demin. | Ad 100% |

## Patentansprüche

1. Quartäre Dialkanolaminester der allgemeinen Formel (I) 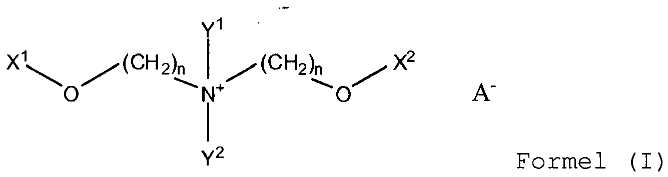 mit
Y¹ und Y² unabhängig voneinander, gleich oder verschieden ein Alkylrest mit 1 bis 6 Kohlenstöffatomen, ein Benzylrest oder H,
n = 1 bis 4
A- ein Anion, bevorzugt ausgewählt aus der Gruppe enthaltend Chlorid, Bromid, Methylsulfat, Tosylat, Phosphat, Sulfat, Hydrogensulfat, Lactat, Acetat und Citrat,
X¹ und X² unabhängig voneinander, gleich oder verschieden ausgewählt aus den Gruppen (a), (b), (c) und (d), wobei diese Gruppen bestehen aus
(a) Acylreste enthaltend 6 bis 14 Kohlenstoffatome,
(b) Acylreste enthaltend 15 bis 20 Kohlenstoffatome und mindestens eine Kohlenstoff-Kohlenstoff-Doppelbindung,
(c) Acylreste enthaltend 20 bis 24 Kohlenstoffatome und
(d) Acylreste enthaltend 3 bis 28 Kohlenstoffatome, welche nicht in den Gruppen (a) bis (c) enthalten sind, oder H,
mit der Maßgabe, dass im Zahlenmittel pro Molekül der allgemeinen Formel (I)
0,30 bis 1,50 Reste der Gruppe (a),
0,10 bis 1,30 Reste der Gruppe (b),
0,40 bis 1,60 Reste der Gruppe (c),
0 bis 0,20 Reste der Gruppe (d)
enthalten sind und
die Zahlenmittel pro Molekül der Reste der Gruppen (a), (b), (c) und (d) sich zu 2 addieren.

2. Quartäre Dialkanolaminester gemäß Anspruch 1, **dadurch gekennzeichnet, dass**
Acylreste der Gruppe (a) ausgewählt sind aus C₅H₁₁CO-, C₇H₁₅CO-, C₉H₁₉CO-, C₁₁H₂₃CO- und C₁₃H₂₇CO-,
Acylreste der Gruppe (b) ausgewählt sind aus C₁₅H₂₉CO-, C₁₇H₃₃CO-, C₁₇H₃₁CO-, C₁₇H₂₉CO-, C₁₉H₃₇CO-, C₁₉H₃₁CO- und C₁₉H₂₉CO-,
Acylreste der Gruppe (c) ausgewählt sind aus C₁₉H₃₉CO-, C₂₁H₄₃CO- und C₂₃H₄₇CO- und
Acylreste der Gruppe (d) ausgewählt sind aus Acylresten der Carbonsäuren, welche zusätzlich in technischen Mischungen der Carbonsäuren enthalten sind, welche den Acylrest der Gruppen (a), (b) und (c) bestimmen.

3. Quartäre Dialkanolaminester gemäß Anspruch 1 oder Anspruch 2, **dadurch gekennzeichnet, dass**
Acylreste der Gruppe (a) ausgewählt sind aus C₁₁H₂₃CO-,
Acylreste der Gruppe (b) ausgewählt sind aus C₁₅H₂₉CO-, C₁₇H₃₃CO, C₁₇H₃,CO-, C₁₇H₂₉CO- und C₁₉H₃₇CO- in einem Gewichtsverhältnis von 3-7 zu 68-76 zu 5-13 zu 1-3 zu 0-2,
Acylreste der Gruppe (c) ausgewählt sind aus C₁₉H₃₉CO-, C₂₁H₄₃CO-, C₂₃H₄₇CO- in einem Gewichtsverhältnis von 4-8 zu 85-99 zu 0-3.

4. Quartäre Dialkanolaminester gemäß mindestens einem der Ansprüche 1 bis 3, **dadurch gekennzeichnet, dass** die Acylreste der Gruppe (a) bis (d) bestimmt werden über die Acylreste einer Mischung bestehend aus technischer Laurinsäure,
technischer Ölsäure und
technischer Behensäure.

5. Quartäre Dialkanolaminester gemäß mindestens einem der Ansprüche 1 bis 4, **dadurch gekennzeichnet, dass** das molare Verhältnis der Summe von Acylresten der Gruppe (a) und der Gruppe (b) zu Acylresten der Gruppe (c) im Zahlenmittel 1 zu 0,67 bis 4 beträgt.

6. Verfahren zur Herstellung quartärer Dialkanolaminester umfassend die Verfahrensschritte
A) Umsetzung eines Alkyldialkanolamins, bei dem die beiden Alkanolgruppen ausgewählt sind aus der Gruppe bestehend aus, -CH₂OH, -C₂H₄OH, -C₃H₆OH und - C₄H₈OH und die Alkylgruppe ausgewählt ist aus der Gruppe bestehend aus Alkylresten mit 1 - 6 Kohlenstoffatomen
mit einem Gemisch aus Carbonsäuren bestehend aus den Gruppen
(e) Carbonsäure enthaltend 6 bis 14 Kohlenstoffatome
(f) Carbonsäure enthaltend 15 bis 20 Kohlenstoffatome und mindestens eine Kohlenstoff-Kohlenstoff-Doppelbindung und
(g) Carbonsäure enthaltend 20 bis 24 Kohlenstoffatome und gegebenenfalls
(h) Carbonsäure enthaltend 3 bis 28 Kohlenstoffatome, welche nicht in den Gruppen (e), (f) und (g) enthalten sind,
B) Umsetzung des Produktes aus Verfahrensschritt A) mit einem Quaternierungsmittel ausgewählt aus der Gruppe umfassend Dialkylsulfate, Alkylhalogenide und Benzylhalogenide und/oder
C) Neutralisierung des Reaktionsansatzes mit einer Säure,
mit der Maßgabe, dass bezogen auf alle Carbonsäuren der Gruppen (e), (f), (g) und (h) 0 bis 10 Mol% Carbonsäuren der Gruppe (h) eingesetzt werden.

7. Verfahren gemäß Anspruch 6, **dadurch gekennzeichnet, dass** in Verfahrensschritt A) pro mol eingesetztem Alkyldialkanolamin
0,30 bis 1,50 mol Carbonsäure der Gruppe (e)
0,10 bis 1,30 mol Carbonsäure der Gruppe (f) und
0,40 bis 1,60 mol Carbonsäure der Gruppe (g)
eingesetzt werden.

8. Verfahren gemäß Anspruch 6 oder 7, **dadurch gekennzeichnet, dass** pro mol Alkyldialkanolamin insgesamt Carbonsäuren der Gruppen (e), (f), (g) und gegebenenfalls (h) in einer Menge von 1,80 bis 2,5 mol eingesetzt werden.

9. Verfahren gemäß mindestens einem der Ansprüche 6 bis 8, **dadurch gekennzeichnet, dass** in Verfahrensschritt B) mindestens ein Lösungsmittel ausgewählt aus der Gruppe bestehend aus kurzkettigen Alkoholen, Fettalkoholen, Polyolen und Carbonaten
eingesetzt wird.

10. Zusammensetzung enthaltend
quartäre Dialkanolaminester gemäß mindestens einem der Ansprüche 1 bis 5 und/oder
quartäre Dialkanolaminester erhältlich nach einem Verfahren gemäß mindestens einem der Ansprüche 6 bis 9,
mindestens ein Lösungsmittel und optional
mindestens eine Carbonsäure ausgewählt aus mindestens einer der Gruppen (e), (f), (g) und (h),
wobei vorzugsweise das mindestens eine Lösungsmittel 0,5 Gel.-% bis 50 Gew.-%, bezogen auf das Gesamtgewicht der Zusammensetzung ausmacht.

11. Zusammensetzung gemäß Anspruch 10, **dadurch gekennzeichnet, dass** das Lösungsmittel Isopropanol oder Propylencarbonat ist.

12. Verwendung quartärer Dialkanolaminester gemäß mindestens einem der Ansprüche 1 bis 5 und/oder quartärer Dialkanolaminester erhältlich nach einem Verfahren gemäß mindestens einem der Ansprüche 6 bis 9 und/oder mindestens einer Zusammensetzung gemäß Anspruch 10 oder 11 zur Herstellung von Formulierungen, insbesondere von kosmetischen oder pharmazeutischen Formulierungen und Pflege- und Reinigungsformulierungen für die Anwendung im häuslichen und industriellen Umfeld..

13. Formulierung, insbesondere kosmetische oder pharmazeutische Formulierung und Pflege- und Reinigungsformulierung für die Anwendung im häuslichen und industriellen Umfeld, enthaltend quartäre Dialkanolaminester gemäß mindestens einem der Ansprüche 1 bis 5 und/oder quartärer Dialkanolaminester erhältlich nach einem Verfahren gemäß mindestens einem der Ansprüche 6 bis 9 und/oder mindestens eine Zusammensetzung gemäß Anspruch 10 oder 11, insbesondere in einer Menge von 0,1 bis 5 Gew.-%, bevorzugt 0,5 bis 4 Gew.-%, besonders bevorzugt 1 bis 3 Gew.-% bezogen auf die Gesamtformulierung, insbesondere wässrige Formulierung, welche vorzugweise einen pH-Wert von 3,5 bis 5 aufweist.

14. Verwendung quartärer Dialkanolaminester gemäß mindestens einem der Ansprüche 1 bis 5 und/oder quartärer Dialkanolaminester erhältlich nach einem Verfahren gemäß mindestens einem der Ansprüche 6 bis 9 und/oder mindestens einer Zusammensetzung gemäß Anspruch 10 oder 11 und/oder mindestens einer Formulierung gemäß Anspruch 13 als Pflegemittel, insbesondere als Haut- und Haarpflegemittel, und/oder zur Konditionierung von Haaren, und/oder als Weichspüler.

## Claims

1. Quaternary dialkanolamine esters of the general formula (I) 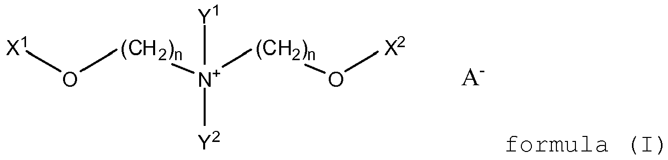 where
Y¹ and Y² are the same or different and are each independently an alkyl radical having 1 to 6 carbon atoms, a benzyl radical or H,
n = 1 to 4
A- is an anion, preferably selected from the group comprising chloride, bromide, methylsulfate, tosylate, phosphate, sulfate, hydrogensulfate, lactate, acetate and citrate,
X¹ and X² are the same or different and are each independently selected from groups (a), (b), (c) and (d), these groups consisting of
(a) acyl radicals containing 6 to 14 carbon atoms,
(b) acyl radicals containing 15 to 20 carbon atoms and at least one carbon-carbon double bond,
(c) acyl radicals containing 20 to 24 carbon atoms and
(d) acyl radicals which contain 3 to 28 carbon atoms and are not included in groups (a) to (c), or H,
with the proviso that each molecule of the general formula (I) contains a numerical average of
0.30 to 1.50 radicals from group (a),
0.10 to 1.30 radicals from group (b),
0.40 to 1.60 radicals from group (c),
0 to 0.20 radical from group (d),
and
the numerical averages per molecule of the radicals from groups (a), (b), (c) and (d) add up to 2.

2. Quaternary dialkanolamine esters according to Claim 1, **characterized in that**
acyl radicals from group (a) are selected from C₅H₁₁CO-, C₇H₁₅CO-, C₉H₁₉CO-, C₁₁H₂₃CO- and C₁₃H₂₇CO-,
acyl radicals from group (b) are selected from C₁₅H₂₉CO-, C₁₇H₃₃CO-, C₁₇H₃₁CO-, C₁₇H₂₉CO-, C₁₉H₃₇CO-, C₁₉H₃₁CO- and C₁₉H₂₉CO-,
acyl radicals from group (c) are selected from C₁₉H₃₉CO-, C₂₁H₄₃CO- and C₂₃H₄₇CO- and
acyl radicals from group (d) are selected from acyl radicals of the carboxylic acids which are additionally present in technical mixtures of the carboxylic acids which determine the acyl radical from groups (a), (b) and (c).

3. Quaternary dialkanolamine esters according to Claim 1 or Claim 2, **characterized in that**
acyl radicals from group (a) are selected from C₁₁H₂₃CO-,
acyl radicals from group (b) are selected from C₁₅H₂₉CO-, C₁₇H₃₃CO, C₁₇H₃₁CO-, C₁₇H₂₉CO- and C₁₉H₃₇CO- in a weight ratio of 3-7:68-76:5-13:1-3:0-2,
acyl radicals from group (c) are selected from C₁₉H₃₉CO-, C₂₁H₄₃CO-, C₂₃H₄₇CO- in a weight ratio of 4-8:85-99:0-3.

4. Quaternary dialkanolamine esters according to at least one of Claims 1 to 3, **characterized in that** the acyl radicals from groups (a) to (d) are determined via the acyl radicals of a mixture consisting of
technical lauric acid,
technical oleic acid and
technical behenic acid.

5. Quaternary dialkanolamine esters according to at least one of Claims 1 to 4, **characterized in that** the numerical average of the molar ratio of the sum of acyl radicals from group (a) and group (b) to acyl radicals from group (c) is 1:0.67 to 4.

6. Process for preparing quaternary dialkanolamine esters, comprising the process steps of
A) reacting an alkyldialkanolamine in which the two alkanol groups are selected from the group consisting of -CH₂OH, -C₂H₄OH, -C₃H₆OH and -C₄H₈OH and the alkyl group is selected from the group consisting of alkyl radicals having 1-6 carbon atoms
with a mixture of carboxylic acids consisting of the groups of
(e) carboxylic acid containing 6 to 14 carbon atoms
(f) carboxylic acid containing 15 to 20 carbon atoms and at least one carbon-carbon double bond and
(g) carboxylic acid containing 20 to 24 carbon atoms and optionally
(h) carboxylic acid which contains 3 to 28 carbon atoms and is not included in groups (e), (f) and (g),
B) reacting the product from process step A) with a quaternizing agent selected from the group comprising dialkyl sulfates, alkyl halides and benzyl halides and/or
C) neutralizing the reaction mixture with an acid, with the proviso that, based on all carboxylic acids from groups (e), (f), (g) and (h), 0 to 10 mol% of carboxylic acids from group (h) is used.

7. Process according to Claim 6, **characterized in that**, for each mole of alkyldialkanolamine used in process step (A),
0.30 to 1.50 mol of carboxylic acid from group (e)
0.10 to 1.30 mol of carboxylic acid from group (f)
and
0.40 to 1.60 mol of carboxylic acid from group (g) are used.

8. Process according to Claim 6 or 7, **characterized in that**, for each mole of alkyldialkanolamine, carboxylic acids from groups (e), (f), (g) and optionally (h) are used in a total amount of 1.80 to 2.5 mol.

9. Process according to at least one of Claims 6 to 8, **characterized in that**, in process step B) at least one solvent selected from the group consisting of short-chain alcohols, fatty alcohols, polyols and carbonates is used.

10. Composition comprising
quaternary dialkanolamine esters according to at least one of Claims 1 to 5 and/or
quaternary dialkanolamine esters obtainable by a process according to at least one of Claims 6 to 9,
at least one solvent and optionally
at least one carboxylic acid selected from at least one of groups (e), (f), (g) and (h),
the at least one solvent preferably accounting for 0.5% by weight to 50% by weight, based on the total weight of the composition.

11. Composition according to Claim 10, **characterized in that** the solvent is isopropanol or propylene carbonate.

12. Use of quaternary dialkanolamine esters according to at least one of Claims 1 to 5 and/or of quaternary dialkanolamine esters obtainable by a process according to at least one of Claims 6 to 9 and/or of at least one composition according to Claim 10 or 11 for production of formulations, especially of cosmetic or pharmaceutical formulations and care and cleaning formulations for use in the domestic and industrial sector.

13. Formulation, especially cosmetic or pharmaceutical formulation and care and cleaning formulation for use in the domestic and industrial sector, comprising quaternary dialkanolamine esters according to at least one of Claims 1 to 5 and/or quaternary dialkanolamine esters obtainable by a process according to at least one of Claims 6 to 9 and/or at least one composition according to Claim 10 or 11, especially in an amount of 0.1 to 5% by weight, preferably 0.5 to 4% by weight and more preferably 1 to 3% by weight, based on the overall formulation, especially aqueous formulation, which preferably has a pH of 3.5 to 5.

14. Use of quaternary dialkanolamine esters according to at least one of Claims 1 to 5 and/or of quaternary dialkanolamine esters obtainable by a process according to at least one of Claims 6 to 9 and/or of at least one composition according to Claim 10 or 11 and/or of at least one formulation according to Claim 13 as a care composition, especially as a skincare and haircare composition, and/or for conditioning of hair, and/or as a fabric softener.

## Revendications

1. Esters de dialcanolamines quaternaires de formule générale (I) 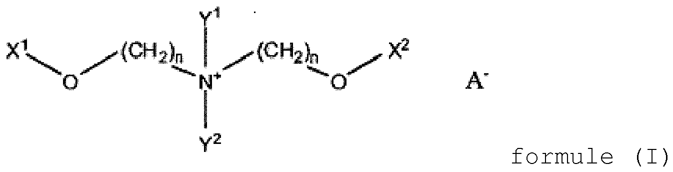 où
Y¹ et Y², identiques ou différents, représentent indépendamment l'un de l'autre un radical alkyle ayant de 1 à 6 atomes de carbone, un radical benzyle ou H,
n = 1 à 4
A représente un anion, choisi de préférence dans le groupe contenant les anions chlorure, bromure, méthylsulfate, tosylate, phosphate, sulfate, hydrogénosulfate, lactate, acétate et citrate,
X¹ et X², identiques ou différents, sont choisis indépendamment l'un de l'autre dans les groupes (a), (b), (c) et (d), ces groupes consistant en
(a) des radicaux acyle comportant de 6 à 14 atomes de carbone,
(b) des radicaux acyle comportant de 15 à 20 atomes de carbone et au moins une double liaison carbone-carbone,
(c) des radicaux acyle comportant de 20 à 24 atomes de carbone et
(d) des radicaux acyle comportant de 3 à 28 atomes de carbone, qui ne sont pas contenus dans les groupes (a) à (c), ou H, étant entendu que sont contenus en moyenne numérique par molécule de formule générale (I)
0,30 à 1,50 radical du groupe (a),
0,10 à 1,30 radical du groupe (b),
0,40 à 1,60 radical du groupe (c),
0 à 0,20 radical du groupe (d)
et
la somme des moyennes numériques des radicaux des groupes (a), (b), (c) et (d) par molécule est égale à 2.

2. Esters de dialcanolamines quaternaires selon la revendication 1, **caractérisés en ce que**
les radicaux acyle du groupe (a) sont choisis parmi C₅H₁₁CO-, C₇H₁₅CO-, C₉H₁₉CO-, C₁₁H₂₃CO- et C₁₃H₂₇CO-,
les radicaux acyle du groupe (b) sont choisis parmi C₁₅H₂₉CO-, C₁₇H₃₃CO-, C₁₇H₃₁CO-, C₁₇H₂₉CO-, C₁₉H₃₇CO-, C₁₉H₃₁CO- et C₁₉H₂₉CO-,
les radicaux acyle du groupe (c) sont choisis parmi C₁₉H₃₉CO-, C₂₁H₄₃CO- et C₂₃H₄₇CO- et
les radicaux acyle du groupe (d) sont choisis parmi des radicaux acyle des acides carboxyliques qui sont en outre contenus dans des mélanges industriels des acides carboxyliques qui déterminent le radical acyle des groupes (a), (b) et (c).

3. Esters de dialcanolamines quaternaires selon la revendication 1 ou la revendication 2, **caractérisés en ce que**
les radicaux acyle du groupe (a) sont choisis parmi C₁₁H₂₃CO-,
les radicaux acyle du groupe (b) sont choisis parmi C₁₅H₂₉CO-, C₁₇H₃₃CO-, C₁₇H₃₁CO-, C₁₇H₂₉CO- et C₁₉H₃₇CO-en un rapport pondéral de 3-7 à 68-76 à 5-13 à 1-3 à 0-2,
les radicaux acyle du groupe (c) sont choisis parmi C₁₉H₃₉CO-, C₂₁H₄₃CO-, C₂₃H₄₇CO- en un rapport pondéral de 4-8 à 85-99 à 0-3.

4. Esters de dialcanolamines quaternaires selon au moins l'une quelconque des revendications 1 à 3, **caractérisés en ce que** les radicaux acyle des groupes (a) à (d) sont déterminés par les radicaux acyle d'un mélange constitué par
l'acide laurique industriel,
l'acide oléique industriel et
l'acide béhénique industriel.

5. Esters de dialcanolamines quaternaires selon au moins l'une quelconque des revendications 1 à 4, **caractérisés en ce que** le rapport molaire de la somme des radicaux acyle du groupe (a) et du groupe (b) aux radicaux acyle du groupe (c) vaut en moyenne numérique 1 : 0,67 à 4.

6. Procédé pour la préparation d'esters de dialcanolamines quaternaires, comprenant les étapes de processus
A) mise en réaction d'une alkyldialcanolamine, dans laquelle les deux groupes alcanol sont choisis dans le groupe constitué par -CH₂OH, -C₂H₄OH, -C₃H₆OH et -C₄H₈OH et le groupe alkyle est choisi dans le groupe constitué par des radicaux alkyle ayant de 1 à 6 atomes de carbone
avec un mélange d'acides carboxyliques consistant en les groupes
(e) acide carboxylique comportant de 6 à 14 atomes de carbone
(f) acide carboxylique comportant de 15 à 20 atomes de carbone et au moins une double liaison carbone-carbone et
(g) acide carboxylique comportant de 20 à 24 atomes de carbone et éventuellement
(h) acide carboxylique comportant de 3 à 28 atomes de carbone, qui n'est pas contenu dans les groupes (e), (f) et (g)
B) mise en réaction du produit de l'étape A) du processus avec un agent de quaternisation choisi dans le groupe comprenant des sulfates de dialkyle, halogénures d'alkyle et halogénures de benzyle et/ou
C) neutralisation du mélange réactionnel par un acide,
étant entendu qu'on utilise de 0 à 10 % en moles d'acides carboxyliques du groupe (h) par rapport à tous les acides carboxyliques des groupes (e), (f), (g) et (h) .

7. Procédé selon la revendication 6, **caractérisé en ce que** dans l'étape A) du processus on utilise 0,30 à 1,50 mole d'acide carboxylique du groupe (e) 0,10 à 1,30 mole d'acide carboxylique du groupe (f) et 0,40 à 1,60 mole d'acide carboxylique du groupe (g) par mole d'alkyldialcanolamine utilisée.

8. Procédé selon la revendication 6 ou 7, **caractérisé en ce qu'**on utilise l'ensemble des acides carboxyliques des groupes (e), (f), (g) et éventuellement (h) en une quantité de 1,80 à 2,5 moles par mole d'alkyldialcanolamine

9. Procédé selon au moins l'une quelconque des revendications 6 à 8, **caractérisé en ce que** dans l'étape B) du processus on utilise au moins un solvant choisi dans le groupe constitué par des alcools à courte chaîne, des alcools gras, des polyols et des carbonates.

10. Composition contenant
des esters de dialcanolamines quaternaires selon au moins l'une quelconque des revendications 1 à 5 et/ou
des esters de dialcanolamines quaternaires pouvant être obtenus conformément à un procédé selon au moins l'une quelconque des revendications 6 à 9,
au moins un solvant et en option
au moins un acide carboxylique choisi dans au moins l'un des groupes (e), (f), (g) et (h),
de préférence ledit au moins un solvant représentant 0,5 % en poids à 50 % en poids, par rapport au poids total de la composition.

11. Composition selon la revendication 10, **caractérisée en ce que** le solvant est l'isopropanol ou le carbonate de propylène.

12. Utilisation d'esters de dialcanolamines quaternaires selon au moins l'une quelconque des revendications 1 à 5 et/ou d'esters de dialcanolamines quaternaires pouvant être obtenus conformément à un procédé selon au moins l'une quelconque des revendications 6 à 9 et/ou d'au moins une composition selon la revendication 10 ou 11, pour la préparation de formulations, en particulier de formulations cosmétiques ou pharmaceutiques et de formulations de soin et de nettoyage pour l'application dans le secteur domestique et le secteur industriel.

13. Formulation, en particulier formulation cosmétique ou pharmaceutique et formulation de soin et de nettoyage pour l'application dans le secteur domestique et le secteur industriel, contenant des esters de dialcanolamines quaternaires selon au moins l'une quelconque des revendications 1 à 5 et/ou des esters de dialcanolamines quaternaires pouvant être obtenus conformément à un procédé selon au moins l'une quelconque des revendications 6 à 9 et/ou au moins une composition selon la revendication 10 ou 11, en particulier en une quantité de 0,1 à 5 % en poids, de préférence de 0,5 à 4 % en poids, de façon particulièrement préférée de 1 à 3 % en poids, par rapport à la formulation totale, en particulier formulation aqueuse, qui présente de préférence un pH de 3,5 à 5.

14. Utilisation d'esters de dialcanolamines quaternaires selon au moins l'une quelconque des revendications 1 à 5 et/ou d'esters de dialcanolamines quaternaires pouvant être obtenus conformément à un procédé selon au moins l'une quelconque des revendications 6 à 9 et/ou d'au moins une composition selon la revendication 10 ou 11 et/ou d'au moins une formulation selon la revendication 13, en tant que produit de soin, en particulier en tant que produit pour le soin de la peau et des cheveux, et/ou pour le conditionnement des cheveux, et/ou en tant qu'assouplissant.
